# EUROPEAN PATENT SPECIFICATION

(11) **EP 4 335 447 B1**
(45) Date of publication and mention of the grant of the patent: **18.06.2025**
(21) Application number: 22827897.4
(22) Date of filing: 01.02.2022
(51) Int. Cl.: A61K 35/32, A61K 35/28, A61P 27/02

(54) **CULTURE SUPERNATANT OF DENTAL PULP-DERIVED STEM CELLS FOR IMPROVING MYOPIA, ASTIGMATISM, PRESBYOPIA, EYE FATIGUE OR BLURRED VISION**
KULTURÜBERSTAND VON AUS ZAHNMARK GEWONNENEN STAMMZELLEN ZUR VERBESSERUNG VON MYOPIE, ASTIGMATISMUS, PRESBYOPIE, AUGENERMÜDUNG ODER VERSCHWOMMENEM SEHEN
SURNAGEANT DE CULTURE DE CELLULES SOUCHES DÉRIVÉES DE LA PULPE DENTAIRE POUR L'AMÉLIORATION DE LA MYOPIE, DE L'ASTIGMATISME, DE LA PRESBYTIE, DE LA FATIGUE OCULAIRE OU DE LA VISION FLOUE

(30) Priority: 21.06.2021 JP 2021102516
(43) Date of publication of application: 13.03.2024
(73) Proprietor: Panagy Co., Ltd., Tokyo, 103-0027 (JP)
(72) Inventor: KOGA Shoji, Tokyo 102-0082 (JP)
(74) Representative: Godemeyer Blum Lenze Patentanwälte Partnerschaft mbB - werkpatent
(86) International application number: PCT/JP2022/003751
(87) International publication number: WO 2022/269973

(56) References cited:
- WO-A1-2011/118795
- WO-A1-2020/130038
- CN-A- 109 419 770
- JP-A- 2015 514 760
- JP-A- 2016 210 730
- JP-A- 2019 156 742
- JP-A- 2019 156 804
- JP-A- 2019 172 607
- EL ZARIF MONA ET AL: "Corneal Stromal Regeneration: A Review of Human Clinical Studies in Keratoconus Treatment", FRONTIERS IN MEDICINE, vol. 8, 23 February 2021 (2021-02-23), FR, XP093122523, ISSN: 2296-858X, DOI: 10.3389/fmed.2021.650724
- JANOWSKI MIROSLAW ET AL: "Concise Review: Using Stem Cells to Prevent the Progression of Myopia-A Concept", STEM CELLS, vol. 33, no. 7, 12 May 2015 (2015-05-12), pages 2104 - 2113, XP093122532, ISSN: 1066-5099, Retrieved from the Internet <URL:https://onlinelibrary.wiley.com/doi/full-xml/10.1002/stem.1984> DOI: 10.1002/stem.1984
- PEI-CHANG WU, CHIA-LING TSAI, GABRIEL M. GORDON, SHINWU JEONG, TATSUO ITAKURA, NITIN PATEL, SONGTAO SHI, M. ELIZABETH FINI: "Chondrogenesis in scleral stem/progenitor cells and its association with form-deprived myopia in mice, ", MOLECULAR VISION, vol. 21, 6 February 2015 (2015-02-06), pages 138 - 147, XP093016695
- Y.-J. YANG, X.-L. LI, Y. XUE, C.-X. ZHANG, Y. WANG, X. HU, Q. DAI : "Bone marrow cells differentiation into organ cells using stem cell therapy", EUROPEAN REVIEW FOR MEDICAL AND PHARMACOLOGICAL SCIENCES, vol. 20, 1 January 2016 (2016-01-01), pages 2899 - 2907, XP093016698

## Description

### Technical Field

The present invention relates to an ocular symptom-improving agent for use in improving an ocular symptom.

### Background Art

With the spread of personal computers, smartphones and the like, the number of people complaining of eye fatigue (also called eyestrain or visual fatigue), blurred vision and associated systemic fatigue (asthenopia) is increasing, and there are growing concerns about an increase in diseases of eyes, such as myopia and astigmatism. Moreover, with the aging of the population, presbyopia due to aging (also called old eyes) is also becoming a social issue. Thus, there is an increasing need for maintenance of the visual function or for prevention, treatment, temporal correction, improvement or the like of ocular symptoms (diseases of eyes; including general troubles of eyes or caused from eyes).

Applications of culture supernatant components obtained by culturing mesenchymal stem cells to ocular symptoms have been known (for example, see PTLs 1 to 3).

PTL 1 describes a pharmaceutical composition for treating at least one of allergic rhinitis and pollinosis which contains an immortalized stem cell culture supernatant containing a secretory component from immortalized stem cells obtained by culturing immortalized stem cells or the secretory component and in which the immortalized stem cells are either dental pulp stem cells or bone marrow stem cells. PTL 1 discloses in Examples that ocular symptoms of pollinosis were improved by administering a culture supernatant using an immortalized dental pulp stem cell culture supernatant to humans as nose drops.

PTL 2 describes a suppressor agent or a therapeutic agent for a cataract containing a culture supernatant component obtained by culturing mesenchymal stem cells. PTL 2 discloses in Examples that cataracts were improved by administering a culture supernatant component using bone marrow mesenchymal stem cells as mesenchymal stem cells to rats as eye drops.

PTL 3 describes a hardening-preventing agent or a therapeutic agent for lens tissues containing a culture supernatant component obtained by culturing mesenchymal stem cells. PTL 3 discloses in Examples that the ability of compressing and recovering of lens tissues was improved by administering a culture supernatant component using bone marrow mesenchymal stem cells as mesenchymal stem cells to rats as eye drops.

El Zarif Mona et al. (Frontiers in Medicine, vol.8, 2021) discloses (cf. abstract) that implantation of autologous adipose-derived adult stems cells were effective in treating patients with advanced keratoconus by corneal stromal regeneration.

### Citation List

### Patent Literature

PTL 1: JP2016-210730A
PTL 2: JP2019-156804A
PTL 3: JP2019-156742A

### Summary of Invention

### Technical Problem

A problem to be solved by the invention is to provide a novel ocular symptom-improving agent related to myopia, astigmatism, presbyopia, eye fatigue or blurred vision. Solution to Problem

As a result of extensive examination to solve the problem, the present inventor has found that a culture supernatant of dental pulp-derived stem cells can improve an ocular symptom related to myopia, astigmatism or presbyopia (in particular, eyesight or astigmatism grade) and can also improve eye fatigue or blurred vision.

Here, in PTL 1, which uses immortalized cells of dental pulp-derived stem cells, improvement of myopia, astigmatism, presbyopia, eye fatigue or blurred vision is not examined.

In PTLs 2 and 3, a culture supernatant of dental pulp-derived stem cells is not examined. Although PTL 3 mentions presbyopia, PTL 3 merely examines that the ability of compressing and recovering of rat lens tissues was improved and fails to examine how far the eyesight of a human patient with presbyopia would be actually improved.

Specifically, the invention and preferable features of the invention are as defined in the claims.

### Advantageous Effects of Invention

According to the invention, a novel ocular symptom-improving agent related to myopia, astigmatism, presbyopia, eye fatigue or blurred vision can be provided.

### Brief Description of Drawings

[Fig. 1] Fig. 1 is a graph showing the relations between the time from the start of the sample administration and the average uncorrected eyesight (calculated with the eyesight before the administration regarded as 100%) of the left and right eyes of patients to whom the sample of Example 1 or Comparative Example 1 was administered.
[Fig. 2] Fig. 2 is a graph showing the relations between the time from the start of the sample administration and the average corrected eyesight (calculated with the eyesight before the administration regarded as 100%) of the left and right eyes of patients to whom the sample of Example 1 or Comparative Example 1 was administered.
[Fig. 3] Fig. 3 is a graph showing the relations between the time from the start of the sample administration and the astigmatism grade of the left eye or the right eye of a patient to whom the sample of Example 1 was administered.
[Fig. 4] Fig. 4 is a graph showing the relations between the time from the start of the sample administration and the astigmatism grade of the left eye or the right eye of another patient to whom the sample of Example 1 was administered.
[Fig. 5] Fig. 5 is a graph showing the relations between the time from the start of the sample administration and the astigmatism grade of the left eye or the right eye of another patient to whom the sample of Example 1 was administered.
[Fig. 6] Fig. 6 is a graph showing the relations between the time from the start of the sample administration and the average uncorrected eyesight (calculated with the eyesight before the administration regarded as 100%) of the left and right eyes at focus distances of 33 cm and 50 cm of patients to whom the sample of Example 1 or Comparative Example 1 was administered.

### Description of Embodiments

The invention is explained in detail below. The constituent features described below are sometimes explained based on typical embodiments or specific examples, but the invention should not be limited to the embodiments. In the description, a numerical range expressed using "to" means a range including the values before and after "to" as the lower limit and the upper limit.

### [Ocular Symptom-Improving Agent]

The ocular symptom-improving agent for use according to the invention is a composition containing a culture supernatant of dental pulp-derived stem cells or immortalized stem cells thereof and is used for improving a symptom of myopia, astigmatism, presbyopia, eye fatigue or blurred vision.

Due to the feature, a novel ocular symptom-improving agent related to myopia, astigmatism, presbyopia, eye fatigue or blurred vision can be provided.

Preferable aspects of the ocular symptom-improving agent for use according to the invention are explained below.

### <Improvement of Ocular Symptom>

In the invention, eye fatigue or blurred vision can be improved, or myopia, astigmatism or presbyopia can be improved. Of these, myopia, astigmatism or presbyopia can be preferably improved, and myopia or presbyopia can be more preferably improved.

The range of the "improvement" in the invention includes not only treatment for completely curing a disease but also temporal treatment or recovery of a disease, even when the disease is not completely cured, stop of the progress of a disease or delay compared to a case without any treatment. Thus, the "improvement of an ocular symptom" in the invention includes not only recovery of the eyesight but also temporal correction of the eyesight and recovery of the eyesight, the vision or the focusing function which is temporarily deteriorated due to eye fatigue or blurred vision and also includes recovery or temporal recovery of the astigmatism grade. For example, a case in which an ocular symptom (in particular, eyesight or astigmatism grade) is temporarily recovered for an administration period of the ocular symptom-improving agent or for a certain period after the completion of the administration and in which the ocular symptom then returns to the original state is also included in the "improvement of an ocular symptom".

The improvement of myopia, astigmatism, presbyopia, eye fatigue or blurred vision is explained below.

### (Improvement of Myopia)

Myopia refers to inability to focus at a far focal point.

Specifically, myopia is caused by deterioration of the ability of the eye to focus and refers to the state in which distant vision for seeing a distant object is difficult. The lens of the eye is a tissue refracting light. To see a distant object, the muscles around the lens pull the lens to reduce the thickness, while to see a close object, the surrounding muscles relax, and the thickness returns to the original thickness due to the elasticity of the lens to focus on the object.

In the case of myopia, light focuses in front of the retina, and thus an object does not appear clearly. Myopia is roughly classified into two types, namely refractive myopia, which is caused because the index of refraction of the cornea or the lens is too strong, and axial myopia, which is caused because the eye's axial length that is the length of the eyeball in the front-back direction is too long.

Refractive myopia refers to the state in which the thickness of the lens playing the role of a lens is not regulated accurately and in which the lens focuses in front of the retina, and refractive myopia is a symptom caused by deterioration of the movement of the lens through temporal paralysis of the ciliary body due to eyestrain (asthenopia), accommodative spasm, tonic accommodation or the like (also called "accommodation disturbance" below). The accommodation disturbance has also been called pseudomyopia, refractive myopia or tonic accommodation myopia, and these are dysfunctions based on transient accommodative spasm or tonic accommodation and are improved by resting the eyes, stopping convulsion of the ciliary body with eye drops to relax the strain of the lens or the like.

Axial myopia refers to the state in which the eye's axial length is long and thus light focuses in front of the retina even when the lens is adjusted thin enough, and it is believed that axial myopia is irreversible and cannot be recovered to the original state. Axial myopia is improved by suppressing the elongation of the eye's axial length, shortening the eye's axial length or the like.

Myopia is caused because focusing especially on a distant object becomes difficult due to these factors.

The "distance" eyesight is believed to be the eyesight at a focal point of one meter or more from the eyes. Here, excellent distance eyesight with naked eyes is also called emmetropia.

The mechanism of development or progress of myopia has not been elucidated sufficiently, and although myopia is corrected with eyeglasses or contact lenses, there is no radical therapy. Even when myopia is corrected with eyeglasses or contact lenses, myopia sometimes progresses. Thus, not only improvement of uncorrected eyesight but also improvement of corrected eyesight in the state with correction with eyeglasses or contact lenses is preferably enabled.

When the ocular symptom-improving agent is used for improving myopia, the ocular symptom-improving agent preferably improves at least one of uncorrected eyesight and corrected eyesight and more preferably improves both of uncorrected eyesight and corrected eyesight.

The uncorrected eyesight is improved preferably by 10% or more based on the eyesight of the subject before the administration regarded as 100%, more preferably by 20% or more, particularly preferably by 30% or more.

The corrected eyesight is improved preferably by 20% or more based on the eyesight of the subject before the administration regarded as 100%, more preferably by 50% or more, particularly preferably by 100% or more.

### (Improvement of Astigmatism)

Astigmatism refers to the inability of the eye to focus on a point object and to create a focused image on the retina.

Astigmatism is visual error or refractive error, and because the eye cannot focus on a point object and create a focused image on the retina, the person has blurred vision. Astigmatism is caused by an abnormal curvature of the cornea, namely irregular curvature or asphericity of the cornea (the curvature of the cornea is not even in all the directions). A perfect cornea is spherical, but the cornea of a person with astigmatism is not spherical. In other words, the cornea is curved more in one direction than in other directions, or the curvature is steep. Thus, the image is not focused on one point but is rather spread.

In astigmatism which can be generally corrected (the state of perpendicular symmetric regular astigmatism), the cornea is distorted in an ellipse shape (approximately a rugby ball shape), and thus the eyesight can be corrected by adding refractive correction strength, namely the cylinder, in the direction with the small curvature with a lens.

In cornea irregular astigmatism in which the cornea is distorted irregularly or in keratoconus with a partial bulge, however, a lens cannot be adopted to the irregularity, and correction or correction of eyesight with eyeglasses is not always possible. Here, keratoconus is a disease in which the cornea bulges for unknown reason.

When the ocular symptom-improving agent is used for improving astigmatism, the ocular symptom-improving agent preferably improves the astigmatism grade (or astigmatic grade) and more preferably improves the astigmatism grade of uncorrected eyesight. The astigmatism grade is improved preferably by 1 or more, more preferably by 2 or more, particularly preferably by 3 or more.

### (Improvement of Presbyopia)

Presbyopia refers to the inability to focus on a near focal point.

Specifically, presbyopia is caused by deterioration of the ability of the eye to focus and refers to the state in which near vision for seeing a close object is difficult. The lens of the eye is a tissue refracting light. To see a distant object, the muscles around the lens pull the lens to reduce the thickness, while to see a close object, the surrounding muscles relax, and the thickness returns to the original thickness due to the elasticity of the lens to focus on the object. With the age, however, the lens hardens and loses the elasticity, or the anterior surface of the lens, which is necessary for shifting the focal point from a distant object to a close object, is curved largely. As a result, focusing especially on a close object is difficult, and presbyopia is developed.

Presbyopia is a normal and inevitable effect of aging, and a first sign generally appears at the age of 40 or later.

In particular, the state with a long minimum focal distance of an individual having excellent distance eyesight with naked eyes is often called presbyopia.

The near eyesight is the eyesight at a focal point of less than one meter from the eyes. Here, the minimum focal distance at which an object is focused is known as the "near point".

When the ocular symptom-improving agent is used for improving presbyopia, the ocular symptom-improving agent preferably improves at least one of the eyesight at a focus distance of 33 cm and the eyesight at a focus distance of 50 cm and more preferably improves both of the eyesight at a focus distance of 33 cm and the eyesight at a focus distance of 50 cm.

The uncorrected eyesight at a focus distance of 33 cm of a subject with old eyes is improved preferably by 10% or more based on the eyesight of the subject before the administration regarded as 100%, more preferably by 20% or more, particularly preferably by 30% or more.

The uncorrected eyesight at a focus distance of 50 cm of a subject with old eyes is improved preferably by 10% or more based on the eyesight of the subject before the administration regarded as 100%, more preferably by 50% or more, particularly preferably by 100% or more.

### (Improvement of Eye Fatigue)

Eye fatigue includes a type caused by overuse of the eyes such as reading, staring operation and observing operation or psychological strain, a type caused by VDT (Visual Display Terminal) operation which is rapidly increasing with the spread of personal computers and the like.

As symptoms of eye fatigue, for example, symptoms such as pain deep in the eyes, neck stiffness and headaches often accompany. Furthermore, in severe eye fatigue, vomiturition or nausea sometimes accompanies. These symptoms are caused because the ciliary muscles are excessively strained due to long staring operation or the like and because the accommodation of the eyes decreases.

Although it is not limited, eye fatigue can be measured using the HFC-1 value and the VAS value measured after one-hour VDT operation as indicators. The HFC-1 value is a value showing the frequency of high-frequency component for staring into a certain distance of the accommodative microfluctuations which are caused when the refractive index of the eyes is continuously recorded. The high-frequency component indicates the vibrations of the lens, namely the vibrations of the ciliary muscles, and a higher frequency of the high-frequency component means that a stronger stress is applied to the ciliary muscles. When the HFC-1 value is high, the ciliary muscles are strained, and the eyes are in fatigue state. The VAS value is a value obtained by measuring, as the severeness of subjective symptom, the length (mm) of the line marked by the panelist as the degree of symptom on a subjective symptom examination sheet with a line of 10 cm, where the panelist checks at 0 mm when the panelist does not feel eyestrain and checks at 100 mm when the panelist feels strong eyestrain, and calculating the value as the eye fatigue score. That is, as the VAS value is higher, the subjective symptom score of eye fatigue is higher.

### (Improvement of Blurred Vision)

Blurred vision refers to a symptom in which the vision blurs due to deterioration of the focusing function of the eyes and in which it takes time to focus when seeing a distant object after seeing a close object. Blurred vision can be caused by overuse of the eyes, inflammation such as a cataract and conjunctivitis, old eyes caused with aging or the like.

### <Mechanisms of Action of Ocular Symptom-Improving Agent>

According to the ocular symptom-improving agent for use according to the invention, myopia and presbyopia, which are decreases in the visual function caused by degeneration of the lens (aging of the eyes, deterioration of the focusing function of the eyes or the like), can be improved. Thus, as a mechanism of action of the ocular symptom-improving agent for use according to the invention, improvement or temporal correction of degeneration of the lens is believed to be possible. Moreover, it is believed that the movement of the axis of the eyeball improves and blurred vision can be improved by improving the focusing function.

According to the ocular symptom-improving agent of the invention, refractive myopia, which is a decrease in the visual function caused by convulsion or strain of the ciliary body or strain or movement of the lens, can be improved. Accordingly, as a mechanism of action of the ocular symptom-improving agent for use according to the invention, improvement of convulsion, strain or the like of the ciliary body due to eye fatigue (eyestrain), asthenopia, accommodative spasm, tonic accommodation or the like, improvement of strain or movement of the lens, improvement of movement of the axis of the eyeball and improvement of refractive myopia are also believed to be possible. That is, that refractive myopia can be improved means that eyestrain is not caused easily, which is believed to mean that eye fatigue can also be improved.

Moreover, as a mechanism of action of the ocular symptom-improving agent for use according to the invention, suppression of elongation of the eye's axial length and suppression of progress of axial myopia are also believed to be possible.

Here, some myotic agents which improve presbyopia only induce an increase in contraction of the ciliary muscles, induce remaining preliminary accommodation, improve near eyesight at the sacrifice of distance eyesight in an individual who still has accommodation function and improve presbyopia only. On the other hand, the ocular symptom-improving agent for use according to the invention is preferable because both myopia and presbyopia can be improved without sacrificing distance eyesight. When the ocular symptom-improving agent of the invention can improve both myopia and presbyopia, according to the various mechanisms of action above, the movement of the axis of the eyeball improves, and eye fatigue and blurred vision are also believed to be able to be improved.

Moreover, according to the ocular symptom-improving agent for use according to the invention, astigmatism can be improved. Thus, as a mechanism of action of the ocular symptom-improving agent for use according to the invention, by making the cornea close to a spherical shape by improving regular distortion, irregular distortion or a partial bulge of the cornea, improvement or temporal correction of regular astigmatism, cornea irregular astigmatism, keratoconus or the like is believed to be possible.

### <Component of Ocular Symptom-Improving Agent>

The ocular symptom-improving agent for use according to the invention is a composition containing a culture supernatant of dental pulp-derived stem cells or immortalized stem cells thereof. The active ingredient of the ocular symptom-improving agent for use according to the invention is a culture supernatant of dental pulp-derived stem cells. The culture supernatant of immortalized stem cells of dental pulp-derived stem cells has a similar active ingredient as that of the culture supernatant of dental pulp-derived stem cells. Preferable aspects of the culture supernatant of dental pulp-derived stem cells are sometimes explained below, and preferable aspects of the culture supernatant of immortalized stem cells of dental pulp-derived stem cells are similar.

The ocular symptom-improving agent for use according to the invention preferably consists of a culture supernatant of dental pulp-derived stem cells or immortalized stem cells.

### (Culture Supernatant of Dental Pulp-Derived Stem Cells)

The culture supernatant of dental pulp-derived stem cells preferably does not substantially contain serum. For example, the serum content of the culture supernatant of dental pulp-derived stem cells is preferably 1 mass% or less, more preferably 0.1 mass% or less, particularly preferably 0.01 mass% or less.

### - Dental Pulp-Derived Stem Cells -

The dental pulp-derived stem cells may be derived from a human or from a nonhuman animal. The nonhuman animal may be the same as the animal (species) as the subject of the administration of the ocular symptom-improving agent for use according to the invention described below and is preferably a mammal.

The dental pulp-derived stem cells used for the culture supernatant are not particularly restricted. Dental pulp stem cells from exfoliated deciduous teeth, deciduous dental pulp stem cells obtained by another method and permanent dental pulp stem cells (DPSCs) can be used.

Somatic stem cells derived from dental pulp can produce vascular endothelial growth factors (VEGFs), hepatocyte growth factors (HGFs), insulin-like growth factors (IGFs), platelet-derived growth factors (PDGFs), transforming growth factors-beta (TGF-β)-1 and -3, TGF-a, KGF, HBEGF, SPARC, other growth factors and various cytokines such as chemokine. Many other bioactive substances such as exosomes can also be produced.

In the invention, the dental pulp-derived stem cells used for the culture supernatant of dental pulp-derived stem cells are particularly preferably dental pulp-derived stem cells containing many proteins, and deciduous dental pulp stem cells are preferably used. That is, in the invention, a culture supernatant of deciduous dental pulp stem cells is preferably used.

The dental pulp-derived stem cells used in the invention may be natural cells or genetically modified cells as long as the intended treatment can be achieved.

In particular, in the invention, immortalized stem cells of dental pulp-derived stem cells can be used. Using immortalized stem cells, which are capable of proliferating substantially infinitely, the amounts and the compositions of the biological factors contained in the culture supernatant of the stem cells can be stabilized over a long period of time. The immortalized stem cells of dental pulp-derived stem cells are not particularly restricted. The immortalized stem cells are preferably non-tumor immortalized stem cells. The immortalized stem cells of dental pulp-derived stem cells can be prepared by adding one of the following small molecule compounds (inhibitors) or any combination thereof to dental pulp-derived stem cells and culturing the cells.

TGFβ receptor inhibitors are not particularly limited as long as the TGFβ receptor inhibitors have the action of inhibiting the function of transforming growth factor (TGF) β receptor, and examples thereof include 2-(5-benzo[1,3]dioxol-4-yl-2-tert-butyl-1H-imidazol-4-yl)-6-methylpyridine, 3-(6-methylpyridin-2-yl)-4-(4-quinolyl)-1-phenylthiocarbamoyl-1H-pyrazole (A-83-01), 2-[(5-chloro-2-fluorophenyl)pteridin-4-yl]pyridin-4-ylamine (SD-208), 3-[(pyridin-2-yl)-4-(4-quinonyl)]-1H-pyrazole and 2-(3-(6-methylpyridin-2-yl)-1H-pyrazol-4-yl)-1,5-naphthyridine (all are from Merck), SB431542 (Sigma-Aldrich) and the like. A-83-01 is preferable.

ROCK inhibitors are not particularly limited as long as the ROCK inhibitors have the action of inhibiting the function of Rho-binding kinase. Examples of the ROCK inhibitors include GSK269962A (Axonmedchem), Fasudil hydrochloride (Tocris Bioscience), Y-27632 and H-1152 (both are from FUJIFILM Wako Pure Chemical Corporation) and the like. Y-27632 is preferable.

GSK3 inhibitors are not particularly limited as long as GSK-3 (glycogen synthase kinase 3) is inhibited, and examples thereof include A 1070722, BIO and BIO-acetoxime (all are from TOCRIS) and the like.

MEK inhibitors are not particularly limited as long as the MEK inhibitors have the action of inhibiting the function of MEK (MAP kinase-ERK kinase), and examples thereof include AZD6244, CI-1040 (PD184352), PD0325901, RDEA119 (BAY86-9766), SL327 and U0126-EtOH (all are from Selleck), PD98059, U0124 and U0125 (all are from Cosmo Bio Co., Ltd.) and the like.

When the ocular symptom-improving agent is used for regenerative medicine, due to the requirements of the Act on the Safety of Regenerative Medicine, the culture supernatant of dental pulp-derived stem cells or immortalized stem cells thereof does not contain other somatic stem cells than the dental pulp-derived stem cells. The ocular symptom-improving agent for use according to the invention may contain mesenchymal stem cells other than the dental pulp-derived stem cells or other somatic stem cells but preferably does not contain such cells.

Examples of the somatic stem cells other than the mesenchymal stem cells include stem cells derived from the dermal system, the digestive system, the myeloid system, the nerve system and the like, but the somatic stem cells are not limited to the examples. Examples of the somatic stem cells of the dermal system include epithelial stem cells, hair follicle stem cells and the like. Examples of the somatic stem cells of the digestive system include pancreatic (general) stem cells, hepatic stem cells and the like. Examples of the somatic stem cells of the myeloid system (except for the mesenchymal stem cells) include hematopoietic stem cells and the like. Examples of the somatic stem cells of the nerve system include neural stem cells, retinal stem cells and the like.

The ocular symptom-improving agent for use according to the invention may contain stem cells other than somatic stem cells but preferably does not contain such stem cells. The stem cells other than somatic stem cells include embryonic stem cells (ES cells), induced pluripotent stem cells (iPS cells) and embryonal carcinoma cells (EC cells).

### - Preparation Method of Culture Supernatant of Dental Pulp-Derived Stem Cells -

The method for preparing the culture supernatant of dental pulp-derived stem cells or immortalized stem cells thereof is not particularly restricted, and a conventional method can be used.

The culture supernatant of dental pulp-derived stem cells is a culture solution obtained by culturing dental pulp-derived stem cells. For example, a culture supernatant which can be used for the invention can be obtained by culturing dental pulp-derived stem cells and then separating and removing the cell components. A culture supernatant which has been appropriately subjected to various treatments (for example, centrifugation, concentration, solvent substitution, dialysis, freezing, drying, lyophilization, dilution, desalination, preservation or the like) may also be used.

The dental pulp-derived stem cells for obtaining the culture supernatant of dental pulp-derived stem cells can be selected by a normal method and can be selected based on the size and the morphology of the cells or as adherent cells. The dental pulp-derived stem cells can be selected from dental pulp cells collected from an exfoliated deciduous tooth or a permanent tooth as adherent cells or passaged cells thereof. The culture supernatant of dental pulp-derived stem cells used can be a culture supernatant obtained by culturing selected stem cells.

The "culture supernatant of dental pulp-derived stem cells" is preferably a culture solution which does not contain the cells themselves obtained by culturing the dental pulp-derived stem cells or the like. The culture supernatant of dental pulp-derived stem cells used in the invention preferably does not contain any cells (regardless of the kinds of cells) as a whole in an aspect. Due to the feature, the composition of the aspect is clearly distinguished of course from the dental pulp-derived stem cells themselves and also from various compositions containing dental pulp-derived stem cells. A typical example of the aspect is a composition which does not contain dental pulp-derived stem cells and which is composed only of a culture supernatant of dental pulp-derived stem cells.

The culture supernatant of dental pulp-derived stem cells used in the invention may contain a culture supernatant of both deciduous dental pulp-derived stem cells and adult dental pulp-derived stem cells. The culture supernatant of dental pulp-derived stem cells used in the invention preferably contains a culture supernatant of deciduous dental pulp-derived stem cells as an active ingredient, more preferably in an amount of 50 mass% or more, preferably 90 mass% or more. The culture supernatant of dental pulp-derived stem cells used in the invention is further particularly preferably a composition composed only of a culture supernatant of deciduous dental pulp-derived stem cells.

A basal medium, a basal medium supplemented with serum and the like or the like can be used for the culture solution of dental pulp-derived stem cells for obtaining the culture supernatant. Examples of the basal medium which can be used include, in addition to Dulbecco's modified Eagle's medium (DMEM), Iscove's modified Dulbecco's medium (IMDM) (GIBCO and the like), Ham's F12 medium (SIGMA, GIBCO and the like), RPMI1640 medium and the like. Examples of the component that can be added to the medium include serum (fetal bovine serum, human serum, sheep serum and the like), serum replacements (knockout serum replacement (KSR) and the like), bovine serum albumin (BSA), antibiotics, various vitamins and various minerals.

In order to prepare "a culture supernatant of dental pulp-derived stem cells" which does not contain serum, however, a serum-free medium should be used throughout the whole process or for the last passaging culture or the last passaging culture steps. For example, when dental pulp-derived stem cells are cultured in a medium which does not contain serum (a serum-free medium), a culture supernatant of dental pulp-derived stem cells which does not contain serum can be prepared. When passaging culture is conducted once or more times and when the last passaging culture or the last passaging culture steps are conducted in a serum-free medium, a culture supernatant of dental pulp-derived stem cells which does not contain serum can also be obtained. Moreover, a culture supernatant of dental pulp-derived stem cells which does not contain serum can also be obtained by removing serum from the recovered culture supernatant using dialysis, solvent substitution using a column or the like.

For culturing the dental pulp-derived stem cells for obtaining the culture supernatant, generally used conditions can be applied as they are. Regarding the method for preparing a culture supernatant of dental pulp-derived stem cells, isolation and selection steps of the stem cells are preferably appropriately adjusted to the type of the stem cells. One skilled in the art can appropriately isolate and select dental pulp-derived stem cells according to the type of the dental pulp-derived stem cells. When the dental pulp-derived stem cells are cultured, the cells are preferably cultured in an incubator, for example at a temperature of 37°C under the conditions of 5% CO₂. Regarding the culture period, the cells are preferably passaged before reaching the confluency. The number of cell passaging of the dental pulp-derived stem cells is not particularly restricted and can be appropriately selected depending on the purpose.

### <Other Components>

The ocular symptom-improving agent for use according to the invention may contain another component in addition to the culture supernatant of dental pulp-derived stem cells depending on the species of the animal as the subject of the administration or the purpose as long as the effects of the invention are not impaired. Examples of the other component include nutritional components, antibiotics, cytokines, protectants, carriers, excipients, disintegrating agents, buffers, pH-adjusting agents, emulsifiers, suspending agents, soothing agents, stabilizers, preservatives, antiseptics and the like. Moreover, the other component may be a component which is generally contained in eye drops.

Examples of the nutritional components include fatty acids, vitamins and the like.

Examples of the antibiotics include penicillin, streptomycin, gentamicin and the like.

The carriers may be materials known as pharmaceutically acceptable carriers.

The ocular symptom-improving agent for use according to the invention may be the culture supernatant itself of dental pulp-derived stem cells or may be a pharmaceutical composition further containing a pharmaceutically acceptable carrier or excipient or the like. The purpose of the pharmaceutical composition is to promote administration of the culture supernatant of dental pulp-derived stem cells to the subject of the administration.

The pharmaceutically acceptable carrier is preferably a carrier (including a diluent) which does not cause significant irritation in the subject of the administration and which does not inhibit the biological activity and the characteristics of the administered composition. Examples of the carrier include propylene glycol, (physiological) saline, an emulsion, a buffer, a medium such as DMEM and RPMI and a low-temperature preservation medium containing a component for removing free radicals.

The ocular symptom-improving agent for use according to the invention may contain the active ingredient of a conventionally known ocular symptom-improving agent. One skilled in the art can appropriately change depending on the use, the subject of the administration and the like.

The ocular symptom-improving agent for use according to the invention preferably does not contain specific substances.

For example, the ocular symptom-improving agent for use according to the invention preferably does not contain dental pulp-derived stem cells. The culture supernatant of dental pulp-derived stem cells is preferably one obtained by removing the dental pulp-derived stem cells from the recovered culture solution. A culture supernatant of dental pulp-derived stem cells from which at least a part of the components which do not contribute to improvement of an ocular symptom, such as the residual medium components (the components remaining in the culture solution after the culture, of the components of the culture solution before the culture) and the water of the culture solution, have been further removed is also included in the culture supernatant of dental pulp-derived stem cells.

Moreover, the ocular symptom-improving agent for use according to the invention may contain MCP-1 or another cytokine. Examples of the other cytokine include those described in [0014] to [0020] of JP2018-023343A and the like.

The ocular symptom-improving agent for use according to the invention may contain Siglec-9 or another sialic acid-binding immunoglobulin-like lectin.

The ocular symptom-improving agent for use according to the invention preferably does not substantially contain serum (fetal bovine serum, human serum, sheep serum or the like). Moreover, the ocular symptom-improving agent for use according to the invention preferably does not substantially contain the conventional serum replacements such as knockout serum replacement (KSR).

The amounts (solid amounts) of the other components in the ocular symptom-improving agent for use according to the invention are each preferably 1 mass% or less, more preferably 0.1 mass% or less, particularly preferably 0.01 mass% or less.

### <Production Method of Ocular Symptom-Improving Agent>

The method for producing the ocular symptom-improving agent for use according to the invention is not particularly restricted.

The ocular symptom-improving agent may be prepared by preparing a culture supernatant of dental pulp-derived stem cells by the above method.

The final form of the ocular symptom-improving agent for use according to the invention is not particularly restricted. For example, the ocular symptom-improving agent for use according to the invention may be in a form in which the culture supernatant of dental pulp-derived stem cells is packed in a container, a form in which the culture supernatant of dental pulp-derived stem cells is solidified by freezing and/or drying and formulated or packed in a container or another form. Examples of the container include a tube, a centrifuge tube, a bag, an eye drop container and the like which are suitable for cryopreservation. The freezing temperature can be, for example, -20°C to -196°C.

### <Use>

The ocular symptom-improving agent is preferably used for administration to a subject with myopia, astigmatism or presbyopia for a therapeutically effective period. The ocular symptom-improving agent is more preferably used for administration to a subject with myopia, astigmatism or presbyopia one or more times a day for a therapeutically effective period.

Moreover, the ocular symptom-improving agent is preferably used for correcting an ocular symptom. The ocular symptom-improving agent is more preferably used for temporarily correcting an ocular symptom of a subject with myopia, astigmatism or presbyopia for an administration period during which the agent is administered to the subject.

The ocular symptom-improving agent is particularly preferably used for temporarily correcting the ocular symptom of the subject for the administration period and for two months after the completion of the administration and is further particularly preferably used for temporarily correcting the ocular symptom of the subject for the administration period and for three months after the completion of the administration.

The ocular symptom-improving agent for use according to the invention contains a culture supernatant of dental pulp-derived stem cells and thus is used also for applications in restorative medicine. In particular, a culture supernatant of dental pulp-derived stem cells is preferably used for applications in restorative medicine. Here, in regenerative medicine based on stem cell transplantation, it is known that stem cells do not play the main role in regeneration and that liquid components produced by the stem cells repair the organ with the autologous stem cells. Difficult problems of the conventional stem cell transplantation, such as malignant transformation, standardization, the administration method, preservation and the culture method, are solved, and restorative medicine is enabled by a culture supernatant of dental pulp-derived stem cells. Compared to stem cell transplantation, the restoration using the culture supernatant of dental pulp-derived stem cells does not easily cause neoplastic transformation or the like and is considered to be safer because cell transplantation is not required. Moreover, the culture supernatant of dental pulp-derived stem cells has advantages because one with uniformly standardized quality can be used. Because mass production and an efficient administration method can be selected, use with a low cost for a wide range of diseases is enabled. The administered culture supernatant of dental pulp-derived stem cells circulates in the body and restores and regenerates any damaged tissue found through activation of the stem cells themselves due to the homing effect. Moreover, the culture supernatant stimulates the pituitary gland, restores the hormone balance and facilitates the metabolic cycle to the original state.

### [Method for Improving Ocular Symptom]

The method for improving an ocular symptom includes administering an effective amount of the ocular symptom-improving agent to a subject with myopia, astigmatism or presbyopia.

### <Administration Method>

The step of administering the ocular symptom-improving agent for use according to the invention to a subject with myopia, astigmatism or presbyopia is not particularly restricted.

The administration method can be application of eye drops or ointment to the eyes, spraying or inhalation to the oral cavity, the nasal cavity or the respiratory tract, infusion, local administration, nasal drops or the like and is preferably little invasive. In the invention, ocular administration to a subject with myopia, astigmatism or presbyopia is preferable.

The number of administrations and the intervals of administration are not particularly restricted. The number of administrations can be one or more times a week and is preferably five times or more, more preferably six times or more, particularly preferably seven times or more. The intervals of administration are preferably an hour to a week, more preferably half a day to a week, particularly preferably a day (once a day) or a higher frequency. However, the number and the intervals can be appropriately adjusted in accordance with the species of the subject of the administration and the symptom of the subject of the administration.

The ocular symptom-improving agent is preferably used for administration to a subject with myopia, astigmatism or presbyopia one or more times a day for a therapeutically effective period. When the subject of the administration is a human, the number of administrations per week is preferably higher, and the agent is preferably administered five times or more a week for a therapeutically effective period or preferably administered every day.

When a culture supernatant of dental pulp-derived stem cells of a general concentration is used, the dose to a human is preferably 0.01 to 5 ml per eye, more preferably 0.02 to 1 ml, further particularly preferably 0.03 to 0.07 ml (about one drop). The dose can be appropriately adjusted in accordance with the symptom of the subject of the administration.

### <Administration Period>

The method for improving an ocular symptom is preferably a method for correcting an ocular symptom (preferably eyesight or astigmatism grade, more preferably eyesight). In the invention, an ocular symptom (preferably eyesight or astigmatism grade, more preferably eyesight) of the subject is preferably temporarily corrected for an administration period during which the ocular symptom-improving agent is administered to the subject.

The therapeutically effective period of the ocular symptom-improving agent for use according to the invention is preferably one month or longer from the start of the administration, preferably two months or longer, particularly preferably three months or longer. During the therapeutically effective period, the degree of improvement of myopia, astigmatism or presbyopia preferably gradually increases. After the degree of improvement of myopia, astigmatism or presbyopia no longer improves (after the therapeutically effective period), an administration period for continuously administering the ocular symptom-improving agent of the invention may be provided, or the administration may be stopped once. By providing an administration period after the therapeutically effective period, the degree of improvement of the ocular symptom can be maintained high for a long period.

After the completion of the administration period, namely after once stopping the administration, the ocular symptom-improving agent may be administered to the subject again according to the need of the subject.

The intermission period between after stopping the administration period and before starting a second or subsequent administration period for administering the ocular symptom-improving agent to the subject again can be appropriately set in accordance with the constitution of the subject, the degree of improvement of the ocular symptom or the need. The intermission period is not particularly restricted. By providing the intermission period after the therapeutically effective period, the degree of improvement of the ocular symptom gradually decreases from an increased state in the intermission period, and the degree of improvement of the ocular symptom (in particular, eyesight or astigmatism grade) sometimes returns to zero.

Here, the period from the completion of the administration period until the degree of improvement of the ocular symptom returns to zero is called an effective correction period. The effective correction period differs with the symptom of the subject, the constitution or the degree of improvement of the ocular symptom. For example, the effective correction period is longer in a subject with presbyopia than a subject with myopia.

The intermission period is preferably until the degree of improvement of the ocular symptom returns to zero. That is, the intermission period is preferably completed during the effective correction period.

In the method for improving an ocular symptom, a second or subsequent administration period for administering the ocular symptom-improving agent to the subject again is preferably repeatedly provided after a predetermined intermission period from the administration period. A second or subsequent administration period for administering the ocular symptom-improving agent to the subject again is more preferably repeatedly provided in the effective correction period from the administration period.

### <Subject of Administration>

The animal (species) as the subject to which the ocular symptom-improving agent for use according to the invention is administered is not particularly restricted. The animal as the subject to which the ocular symptom-improving agent of the invention is administered is preferably a mammal, a bird (chicken, quail, duck or the like) or fish (salmon, trout, tuna, bonito or the like). The mammal may be a human or a nonhuman mammal. The nonhuman mammal is more preferably a cow, a pig, a horse, a goat, a sheep, a monkey, a dog, a cat, a mouse, a rat, a guinea pig or a hamster.

The subject to which the ocular symptom-improving agent for use according to the invention is administered is preferably a human.

The subject of the administration in the method for improving an ocular symptom may be a human or a nonhuman animal, but the subject of the administration is preferably a nonhuman animal.

The ocular symptom-improving agent for use according to the invention may be administered or applied to a subject with an eye disease other than myopia, astigmatism and presbyopia or may be administered or applied to a subject without any other eye disease. In a preferable aspect of the invention, the subject of the administration is preferably a subject without a cataract or glaucoma.

The ocular symptom-improving agent for use according to the invention may be used in combination with a conventionally known ocular symptom-improving agent.

### Examples

The characteristics of the invention are explained further specifically referring to Examples and Comparative Examples below. The materials, the amounts, the proportions, the contents of treatments, the procedures of treatments and the like shown in Examples below can be appropriately changed as long as such changes do not go beyond the intention of the invention. Accordingly, the scope of the invention should not be construed as being limited by the specific examples shown below.

### [Example 1 and Comparative Example 1]

### <Preparation of Culture Supernatant of Dental Pulp-Derived Stem Cells>

A DMEM culture solution was prepared.

According to the method described in Example 6 of JP6296622B except that DMEM medium was used instead of the DMEM/HamF12 mixture medium, human deciduous dental pulp-derived stem cells were seeded to the DMEM culture solution to culture dental pulp-derived stem cells, and the culture supernatant was collected.

The prepared culture supernatant of dental pulp-derived stem cells was used as the ocular symptom-improving sample agent of Example 1.

Moreover, the DMEM culture solution was used as the sample of Comparative Example 1 and used as a control.

### <Evaluation of Improvement of Myopia (1)>

Of 26 adult myopia patients without a cataract and glaucoma, the ocular symptom-improving sample agent of Example 1 was ocularly administered to 18 patients, and the sample of Comparative Example 1 was ocularly administered to eight patients, each once every morning at a dose of one drop (about 0.05 ml) to both eyes. The treatment period was three months after starting the administration.

The eyesight values of the left and right eyes of the patients were measured before the administration, two weeks (2W), one month (1M), two months (2M) and three months (3M) after starting the administration, one month after the completion of the three-month administration (post 1M) and three months after the completion of the administration (post 3M).

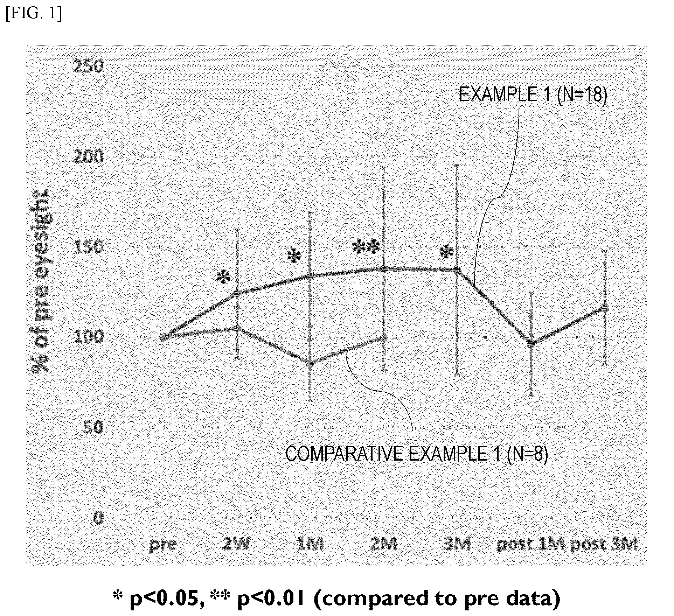

The average uncorrected eyesight values of the left and right eyes of the 18 patients to whom the ocular symptom-improving sample agent of Example 1 was administered were calculated based on the eyesight before the administration (pre eyesight) regarded as 100%. Similarly, the average uncorrected eyesight values of the left and right eyes of the eight patients to whom the sample of Comparative Example 1 was administered were calculated based on the eyesight before the administration regarded as 100%. The obtained results are shown in Fig. 1.

From Fig. 1, it was found that the uncorrected eyesight improved significantly when the ocular symptom-improving sample agent of Example 1 was administered compared to the case in which the sample of Comparative Example 1, which was the DMEM culture solution, was administered. It was also found that the agent is particularly useful for an application for temporarily correcting an ocular symptom (in particular, eyesight) of a subject with myopia for an administration period during which the agent is administered to the subject and for three months after the completion of the administration. Furthermore, it was found that, when the agent is administered to a subject with myopia, the degree of improvement of an ocular symptom is higher in the administration period (immediately before the completion of the administration period) than one month after the completion of the administration (post 1M) or three months after the completion of the administration (post 3M).

### <Evaluation of Improvement of Myopia (2)>

Of the 26 patients whose uncorrected eyesight was measured, the corrected eyesight values of 10 patients with correction with eyeglasses were also measured at the same timings. Specifically, the corrected eyesight values of eight of the patients to whom the ocular symptom-improving sample agent prepared in Example 1 was administered and the corrected eyesight values of two of the patients to whom the sample prepared in Comparative Example 1 was administered were measured.

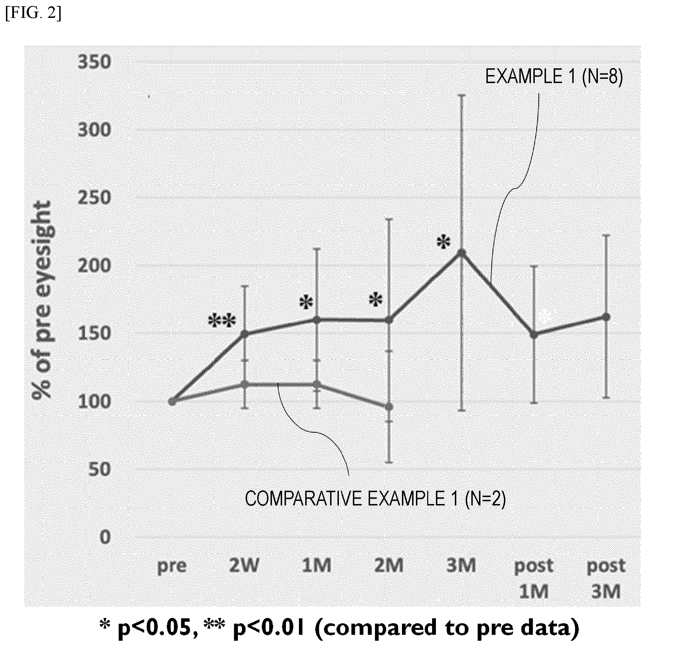

The average corrected eyesight values with eyeglasses of the left and right eyes of the eight patients to whom the ocular symptom-improving sample agent of Example 1 was administered were calculated based on the eyesight before the administration regarded as 100%. Similarly, the average corrected eyesight values with eyeglasses of the left and right eyes of the two patients to whom the sample of Comparative Example 1 was administered were calculated based on the eyesight before the administration regarded as 100%. The obtained results are shown in Fig. 2.

From Fig. 2, it was found that the corrected eyesight improved significantly when the ocular symptom-improving sample agent of Example 1 was administered compared to the case in which the sample of Comparative Example 1, which was the DMEM culture solution, was administered. From Fig. 2, it was also found that the agent is particularly useful for an application for temporarily correcting an ocular symptom (in particular, eyesight) of a subject with myopia for an administration period during which the agent is administered to the subject and for three months after the completion of the administration. Furthermore, it was found that, when the agent is administered to a subject with myopia, the degree of improvement of an ocular symptom is higher in the administration period (immediately before the completion of the administration period) than one month after the completion of the administration (post 1M) or three months after the completion of the administration (post 3M).

From the above results, it was found that the ocular symptom-improving agent of the invention can improve myopia and can improve both uncorrected eyesight and corrected eyesight.

### [Example 2]

### <Evaluation of Improvement of Astigmatism>

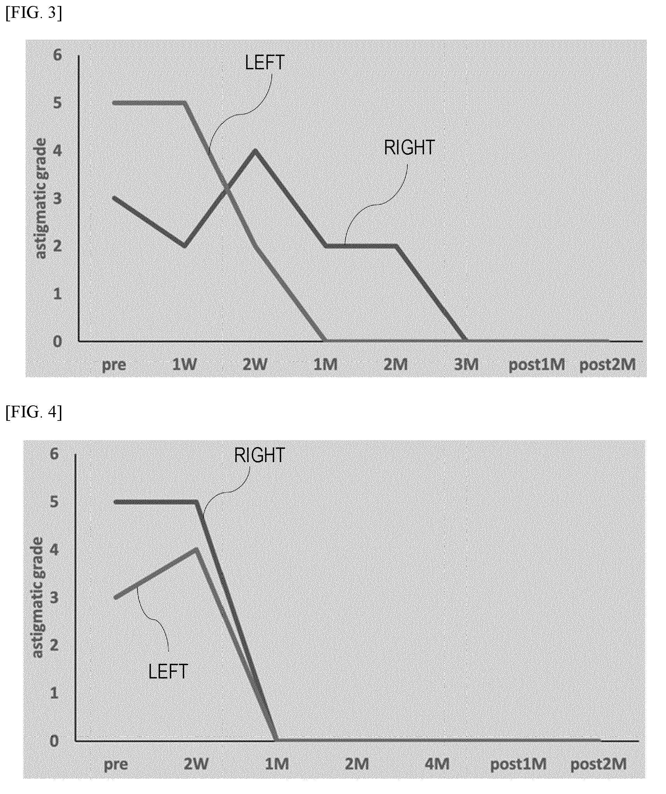

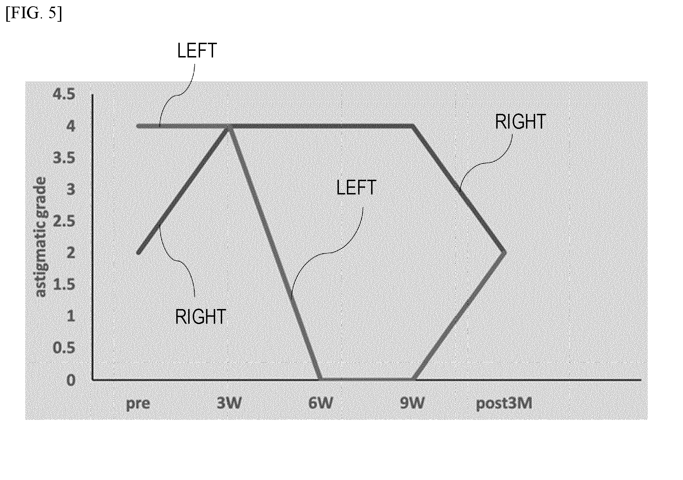

Of the 26 patients whose uncorrected eyesight was measured, the astigmatism grades (or the astigmatic grades) of three patients with astigmatism were also measured at the same timings. Specifically, the astigmatism grades with naked eyes of three of the patients to whom the ocular symptom-improving sample agent prepared in Example 1 was administered were measured. The obtained results of the right eyes and the left eyes of the patients are shown in Figs. 3 to 5.

From Figs. 3 to 5, it was found that astigmatism improved when the ocular symptom-improving sample agent of Example 1 was administered. From Figs. 3 to 5, it was also found that the agent is particularly useful for an application for temporarily correcting an ocular symptom (in particular, astigmatism grade) of a subject with astigmatism for an administration period during which the agent is administered to the subject and for two months after the completion of the administration. Furthermore, it was found that, when the agent is administered to a subject with astigmatism, the degree of improvement of astigmatism in the administration period (immediately before the completion of the administration period) is equivalent to that one month after the completion of the administration (post 1M) or two months after the completion of the administration (post 2M).

### [Example 3]

### <Evaluation of Improvement of Presbyopia>

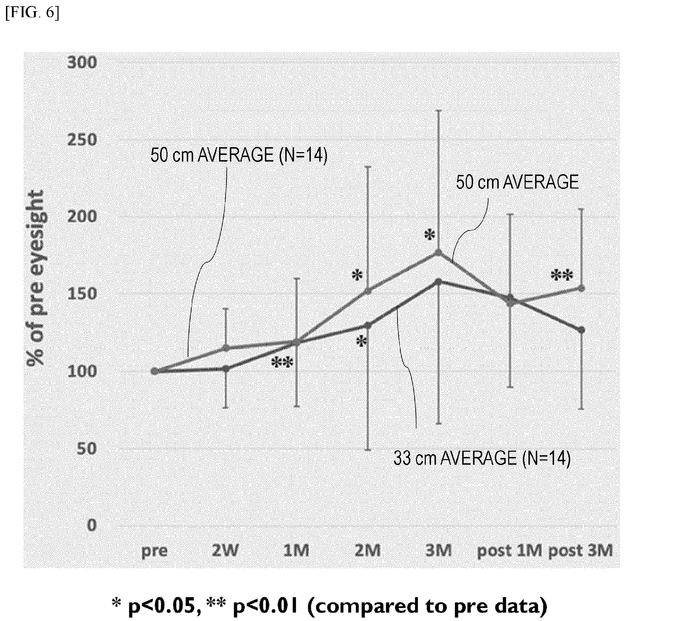

Of the 26 patients whose uncorrected eyesight was measured, presbyopia of 14 patients with presbyopia at the age of 40 or older was also evaluated at the same timings. Specifically, the eyesight values at focus distances of 33 cm and 50 cm with naked eyes of 14 of the patients to whom the ocular symptom-improving sample agent prepared in Example 1 was administered were measured. The average uncorrected eyesight values of the left and right eyes were calculated based on the eyesight before the administration (pre eyesight) regarded as 100%. The obtained results are shown in Fig. 6.

From Fig. 6, it was found that presbyopia improved significantly when the ocular symptom-improving sample agent of Example 1 was administered. That is, it was found that the ocular symptom-improving agent of the invention can improve presbyopia and can improve both of the eyesight at a focus distance of 33 cm and the eyesight at a focus distance of 50 cm. From Fig. 6, it was also found that the agent is particularly useful for an application for temporarily correcting an ocular symptom (in particular, eyesight) of a subject with presbyopia for an administration period during which the agent is administered to the subject and for three months after the completion of the administration. Furthermore, it was found that, when the agent is administered to a subject with presbyopia, the degree of improvement of an ocular symptom is higher in the administration period (immediately before the completion of the administration period) than one month after the completion of the administration (post 1M) or three months after the completion of the administration (post 3M).

Here, it was found that, compared to the results of temporal correction of the eyesight of the subjects with myopia shown in Fig. 1 and Fig. 2, the way of returning of the degree of improvement of the eyesight one month after the completion of the administration (post 1M) or three months after the completion of the administration (post 3M) was milder in the case of temporal correction of the eyesight of the subjects with presbyopia shown in Fig. 6. That is, it was found that the effect of the ocular symptom-improving agent (temporal correction of eyesight) lasts longer with the administration to a subject with presbyopia than with the administration to a subject with myopia. Accordingly, it was found that, especially when the eyesight of a subject with presbyopia is temporarily corrected, the ocular symptom-improving agent of the invention is extremely useful for an application for temporarily correcting an ocular symptom (in particular, eyesight) of a subject with presbyopia for an administration period during which the agent is administered to the subject and for three months after the completion of the administration.

As shown in Fig. 1, Fig. 2 and Fig. 6, the ocular symptom-improving agent of the invention can improve both myopia and presbyopia and is thus believed to be able to improve convulsion or strain of the ciliary body, strain or movement of the lens, degeneration of the lens or the focusing function of the eyes and improve the movement of the axis of the eyeball, considering the mechanisms of action. When convulsion or strain of the ciliary body, strain or movement of the lens, degeneration of the lens or the focusing function of the eyes can be improved and when the movement of the axis of the eyeball improves, eye fatigue and blurred vision are believed to be further improved.

A culture supernatant of adipose-derived stem cells was prepared in the same manner as in Example 1 (Preparation of Culture Supernatant of Dental Pulp-Derived Stem Cells) in 0054 of the description of the present application except for using adipose-derived stem cells.

### (1) Evaluation of Improvement of Myopia

The ocular symptom-improving sample agent of Example 1 (the culture supernatant of dental pulp-derived stem cells) was ocularly administered to the right eyes of three adult myopia patients without a cataract and glaucoma (patient A aged 25 years, patient B aged 30 years and patient C aged 50 years), and a sample of Comparative Example A (the culture supernatant of adipose-derived stem cells) was ocularly administered to the left eyes, each once every morning at a dose of one drop (about 0.05 ml). The treatment period was three months after starting the administration.

The eyesight values of the naked left and right eyes of the three patients were measured before the administration (pre), one month (1M), two months (2M) and three months (3M) after starting the administration.

Moreover, for patient A aged 25 years and patient B aged 30 years who wore contact lenses, the corrected eyesight values of the left and right eyes were similarly measured.

The uncorrected eyesight values or the corrected eyesight values of the right eyes and the left eyes of the patients were calculated based on the eyesight before the administration (pre eyesight) regarded as 100%. The obtained results are shown in the tables below.

**[Table 1]**

| 25 Years Old (Uncorrected) (% of pre eyesight) | | pre | 1M | 2M | 3M |
|---|---|---|---|---|---|
| Culture Supernatant of Dental Pulp-Derived Stem Cells | Right Eye | 100 | 140 | 200 | 200 |
| Culture Supernatant of Adipose-Derived Stem Cells | Left Eye | 100 | 100 | 100 | 100 |

**[Table 2]**

| 25 Years Old (Corrected) (% of pre eyesight) | | pre | 1M | 2M | 3M |
|---|---|---|---|---|---|
| Culture Supernatant of Dental Pulp-Derived Stem Cells | Right Eye | 100 | 67 | 100 | 117 |
| Culture Supernatant of Adipose-Derived Stem Cells | Left Eye | 100 | 89 | 89 | 89 |

**[Table 3]**

| 30 Years Old (Uncorrected) (% of pre eyesight) | | pre | 1M | 2M | 3M |
|---|---|---|---|---|---|
| Culture Supernatant of Dental Pulp-Derived Stem Cells | Right Eye | 100 | 100 | 200 | 200 |
| Culture Supernatant of Adipose-Derived Stem Cells | Left Eye | 100 | 100 | 200 | 100 |

**[Table 4]**

| 30 Years Old (Corrected) (% of pre eyesight) | | pre | 1M | 2M | 3M |
|---|---|---|---|---|---|
| Culture Supernatant of Dental Pulp-Derived Stem Cells | Right Eye | 100 | 150 | 150 | 150 |
| Culture Supernatant of Adipose-Derived Stem Cells | Left Eye | 100 | 150 | 125 | 125 |

**[Table 5]**

| 50 Years Old (Uncorrected) (% of pre eyesight) | | pre | 1M | 2M | 3M |
|---|---|---|---|---|---|
| Culture Supernatant of Dental Pulp-Derived Stem Cells | Right Eye | 100 | 400 | 800 | 800 |
| Culture Supernatant of Adipose-Derived Stem Cells | Left Eye | 100 | 100 | 100 | 100 |

From the tables above, it was found that myopia improved significantly when the ocular symptom-improving sample agent of Example 1 (the culture supernatant of dental pulp-derived stem cells) was administered compared to the case in which the sample of Comparative Example 1, which was the culture supernatant of adipose-derived stem cells, was administered.

### (2) Evaluation of Improvement of Presbyopia

For patient B aged 30 years and patient C aged 50 years who also had presbyopia, of the three patients, the eyesight values (uncorrected eyesight for patient B aged 30 years, and corrected eyesight for patient C aged 50 years) at a focus distance of 33 cm of the left and right eyes were similarly measured. The uncorrected eyesight values or the corrected eyesight values of the right eyes and the left eyes of the patients were calculated based on the eyesight before the administration (pre eyesight) regarded as 100%. The obtained results are shown in the tables below.

**[Table 6]**

| 30 Years Old (Uncorrected) Focus Distance of 33 cm (% of pre eyesight) | | pre | 1M | 2M | 3M |
|---|---|---|---|---|---|
| Culture Supernatant of Dental Pulp-Derived Stem Cells | Right Eye | 100 | 300 | 400 | 600 |
| Culture Supernatant of Adipose-Derived Stem Cells | Left Eye | 100 | 150 | 100 | 150 |

**[Table 7]**

| 50 Years Old (Corrected) Focus Distance of 33 cm (% of pre eyesight) | | pre | 1M | 2M | 3M |
|---|---|---|---|---|---|
| Culture Supernatant of Dental Pulp-Derived Stem Cells | Right Eye | 100 | 100 | 120 | 150 |
| Culture Supernatant of Adipose-Derived Stem Cells | Left Eye | 100 | 100 | 114.2857 | 114 |

From the tables above, it was found that presbyopia improved significantly when the ocular symptom-improving sample agent of Example 1 (the culture supernatant of dental pulp-derived stem cells) was administered compared to the case in which the sample of Comparative Example 1, which was the culture supernatant of adipose-derived stem cells, was administered.

## Claims

1. An ocular symptom-improving agent
which is a composition containing a culture supernatant of dental pulp-derived stem cells or a culture supernatant of immortalized stem cells of dental pulp-derived stem cells for use in improving myopia, astigmatism or presbyopia.

2. The ocular symptom-improving agent for use according to claim 1 which consists of the culture supernatant of dental pulp-derived stem cells or the culture supernatant of immortalized stem cells.

3. The ocular symptom-improving agent for use according to claim 1 or 2 which is for use in improving myopia and which improves at least one of uncorrected eyesight and corrected eyesight.

4. The ocular symptom-improving agent for use according to claim 1 or 2 which is for use in improving presbyopia and which improves at least one of the eyesight at a focus distance of 33 cm and the eyesight at a focus distance of 50 cm.

5. The ocular symptom-improving agent for use according to any one of claims 1 to 4 which is used for ocular administration of the ocular symptom-improving agent to a subject with myopia, astigmatism or presbyopia.

6. The ocular symptom-improving agent for use according to claim 5, wherein the subject is a human.

7. The ocular symptom-improving agent for use according to claim 5 or 6, wherein the subject is a subject without a cataract or glaucoma.

8. The ocular symptom-improving agent for use according to any one of claims 5 to 7 which is used for administration of the ocular symptom-improving agent to the subject one or more times a day for a therapeutically effective period.

9. The ocular symptom-improving agent for use according to any one of claims 1 to 8
in which the ocular symptom-improving agent is for use in correcting an ocular symptom and
which is for use in temporarily correcting an ocular symptom of a subject with myopia, astigmatism or presbyopia for an administration period during which the ocular symptom-improving agent is administered to the subject.

10. The ocular symptom-improving agent for use according to claim 9 which is for use in temporarily correcting the ocular symptom of the subject for the administration period and for three months after the completion of the administration.

11. The ocular symptom-improving agent for use according to claim 1, wherein the subject is a nonhuman animal.

12. The ocular symptom-improving agent for use according to claim 1 in which a second or subsequent administration period for administering the ocular symptom-improving agent to the subject again is repeatedly provided after a predetermined period from the administration period.

13. Non-therapeutic use of an ocular symptom-improving agent
which is a composition containing a culture supernatant of dental pulp-derived stem cells or a culture supernatant of immortalized stem cells of dental pulp-derived stem cells for improving eye fatigue or blurred vision.

## Patentansprüche

1. Ein Mittel zur Verbesserung von Augensymptomen, das eine Zusammensetzung ist, die einen Kulturüberstand von aus Zahnpulpa gewonnenen Stammzellen oder einen Kulturüberstand von immortalisierten Stammzellen von aus Zahnpulpa gewonnenen Stammzellen enthält, zur Verwendung zur Verbesserung von Myopie, Astigmatismus oder Alterssichtigkeit.

2. Das Mittel zur Verbesserung von Augensymptomen zur Verwendung gemäß Anspruch 1, das aus dem Kulturüberstand von aus Zahnpulpa gewonnenen Stammzellen oder dem Kulturüberstand von immortalisierten Stammzellen besteht.

3. Das Mittel zur Verbesserung von Augensymptomen zur Verwendung gemäß Anspruch 1 oder 2, das zur Verbesserung der Myopie verwendet wird und das mindestens eines von unkorrigiertem Sehvermögen und korrigiertem Sehvermögen verbessert.

4. Das Mittel zur Verbesserung von Augensymptomen zur Verwendung gemäß Anspruch 1 oder 2, das zur Verbesserung der Alterssichtigkeit verwendet wird und das mindestens eines von der Sehkraft bei einer Fokusentfernung von 33 cm und der Sehkraft bei einer Fokusentfernung von 50 cm verbessert.

5. Das Mittel zur Verbesserung von Augensymptomen zur Verwendung gemäß einem der Ansprüche 1 bis 4, das zur okularen Verabreichung des Mittels zur Verbesserung der Augensymptome an ein Subjekt mit Myopie, Astigmatismus oder Alterssichtigkeit verwendet wird.

6. Das Mittel zur Verbesserung von Augensymptomen zur Verwendung gemäß Anspruch 5, wobei es sich bei dem Subjekt um einen Menschen handelt.

7. Das Mittel zur Verbesserung von Augensymptomen zur Verwendung gemäß Anspruch 5 oder 6, wobei es sich bei dem Subjekt um ein Subjekt ohne Katarakt oder Glaukom handelt.

8. Das Mittel zur Verbesserung von Augensymptomen zur Verwendung gemäß einem der Ansprüche 5 bis 7, das zur Verabreichung des Mittels zur Verbesserung der Augensymptome an das Subjekt ein- oder mehrmals täglich über einen therapeutisch wirksamen Zeitraum verwendet wird.

9. Das Mittel zur Verbesserung von Augensymptomen zur Verwendung gemäß einem der Ansprüche 1 bis 8,
wobei das Mittel zur Verbesserung von Augensymptomen zur Korrektur eines Augensymptoms verwendet wird, und
das zur vorübergehenden Korrektur eines Augensymptoms eines Subjekts mit Myopie, Astigmatismus oder Alterssichtigkeit für einen Verabreichungszeitraum verwendet wird, während dem dem Subjekt das Mittel zur Verbesserung der Augensymptome verabreicht wird.

10. Das Mittel zur Verbesserung von Augensymptomen zur Verwendung gemäß Anspruch 9, das zur vorübergehenden Korrektur des Augensymptoms des Subjekts während des Verabreichungszeitraums und für drei Monate nach Abschluss der Verabreichung verwendet wird.

11. Das Mittel zur Verbesserung von Augensymptomen zur Verwendung gemäß Anspruch 1, wobei das Subjekt ein nichtmenschliches Tier ist.

12. Das Mittel zur Verbesserung von Augensymptomen zur Verwendung gemäß Anspruch 1, bei dem nach einer vorbestimmten Zeitspanne ab der Verabreichungsperiode wiederholt eine zweite oder nachfolgende Verabreichungsperiode zur erneuten Verabreichung des Mittels zur Verbesserung von Augensymptomen an das Subjekt vorgesehen ist.

13. Nicht-therapeutische Verwendung eines Mittels zur Verbesserung von Augensymptomen, das eine Zusammensetzung ist, die einen Kulturüberstand von aus Zahnpulpa gewonnenen Stammzellen oder einen Kulturüberstand von immortalisierten Stammzellen von aus Zahnpulpa gewonnenen Stammzellen enthält, zur Verbesserung von Augenermüdung oder verschwommenem Sehen.

## Revendications

1. Agent permettant d'améliorer des symptômes oculaires
qui est une composition contenant un surnageant de culture de cellules souches dérivées de pulpe dentaire ou un surnageant de culture de cellules souches immortalisées de cellules souches dérivées de pulpe dentaire à utiliser pour améliorer la myopie, l'astigmatisme ou la presbytie.

2. Agent permettant d'améliorer des symptômes oculaires pour une utilisation selon la revendication 1, qui consiste en le surnageant de culture de cellules souches dérivées de pulpe dentaire ou le surnageant de culture de cellules souches immortalisées.

3. Agent permettant d'améliorer des symptômes oculaires pour une utilisation selon la revendication 1 ou 2, qui est destiné à une utilisation permettant d'améliorer la myopie et qui améliore au moins l'une des deux valeurs suivantes : la vue non corrigée et la vue corrigée.

4. Agent permettant d'améliorer des symptômes oculaires pour une utilisation selon la revendication 1 ou 2, qui est destiné à une utilisation permettant d'améliorer la presbytie et qui améliore au moins l'une des deux valeurs suivantes : la vue à une distance de mise au point de 33 cm et la vue à une distance de mise au point de 50 cm.

5. Agent permettant d'améliorer des symptômes oculaires pour une utilisation selon l'une quelconque des revendications 1 à 4 qui est utilisé pour l'administration oculaire de l'agent permettant d'améliorer des symptômes oculaires à un sujet atteint de myopie, d'astigmatisme ou de presbytie.

6. Agent permettant d'améliorer des symptômes oculaires à utiliser selon la revendication 5, dans lequel le sujet est un humain.

7. Agent permettant d'améliorer des symptômes oculaires pour une utilisation selon la revendication 5 ou 6, dans lequel le sujet est un sujet sans cataracte ni glaucome.

8. Agent permettant d'améliorer des symptômes oculaires pour une utilisation selon l'une quelconque des revendications 5 à 7 qui est utilisé pour l'administration de l'agent permettant d'améliorer des symptômes oculaires au sujet une ou plusieurs fois par jour pendant une période thérapeutiquement efficace.

9. Agent permettant d'améliorer des symptômes oculaires pour une utilisation selon l'une quelconque des revendications 1 à 8 dans lequel l'agent permettant d'améliorer des symptômes oculaires pour une utilisation pour corriger un symptôme oculaire et qui est destiné à une utilisation pour corriger temporairement un symptôme oculaire chez un sujet atteint de myopie, d'astigmatisme ou de presbytie pendant une période d'administration au cours de laquelle l'agent permettant d'améliorer des symptômes oculaires est administré au sujet.

10. Agent permettant d'améliorer des symptômes oculaires selon la revendication 9, destiné à une utilisation pour corriger temporairement le symptôme oculaire du sujet pendant la période d'administration et pendant trois mois après la fin de l'administration.

11. Agent permettant d'améliorer des symptômes oculaires pour une utilisation selon la revendication 1, dans lequel le sujet est un animal non humain.

12. Agent permettant d'améliorer des symptômes oculaires pour une utilisation selon la revendication 1, dans lequel une deuxième période d'administration ou une période d'administration ultérieure pour administrer à nouveau l'agent permettant d'améliorer des symptômes oculaires au sujet est fournie de manière répétée après une période prédéterminée à partir de la période d'administration.

13. Utilisation non thérapeutique d'un agent permettant d'améliorer des symptômes oculaires qui est une composition contenant un surnageant de culture de cellules souches dérivées de pulpe dentaire ou un surnageant de culture de cellules souches immortalisées de cellules souches dérivées de pulpe dentaire pour améliorer la fatigue oculaire ou la vision floue.
